# EUROPEAN PATENT APPLICATION

(11) **EP 0 700 929 A2**
(43) Date of publication of application: **13.03.1996**
(21) Application number: 95306295.7
(22) Date of filing: 08.09.1995
(51) Int. Cl.: C07K 14/415, A61K 39/36

(54) **Peptide derived from cedar pollen allergens and uses thereof**

(30) Priority: 10.09.1994 JP 242137/94; 14.07.1995 JP 200204/95; 14.07.1995 JP 200221/95
(71) Applicant: KABUSHIKI KAISHA HAYASHIBARA SEIBUTSU KAGAKU KENKYUJO, Okayama-shi Okayama (JP)
(72) Inventor: Saito, Saburo, Shinagawa-ku, Tokyo (JP); Hino, Katsuhiko, Okayama-shi, Okayama (JP); Taniguchi, Yoshifumi, Okayama-shi, Okayama (JP)
(74) Representative: Daniels, Jeffrey Nicholas

(57) **Abstract**

Disclosed are peptides which essentially consist of T-cell epitope(s) of cedar pollen allergens, homologous peptides thereunto, and immunotherapeutic agents containing such peptides as an effective ingredient. These peptides do not substantially react with immunoglobulin E antibody specific to cedar pollen allergens, and significantly more activate T-cells specific to cedar pollen allergens than a negative control when assayed by the ³H-thymidine uptake method. Thus, the agents can effectively treat and/or the prevent cedar pollinosis without fear of causing side effects when administered to mammals including human.

SEQUENCE LISTING

(1) GENERAL INFORMATION:

(i) APPLICANT:
NAME:KABUSHIKI KAISHA HAYASHIBARA SEIBUTSU KAGAKU
KENKYUJO

(ii) TITLE OF INVENTION:PEPTIDE AND USES OF THE SAME

(iii) NUMBER OF SEQUENCES:24

(iv) ADDRESS:
(A) ADDRESSEE:KABUSHIKI KAISHA HAYASHIBARA SEIBUTSU
KAGAKU KENKYUJO
(B) STREET:2-3, 1-CHOME, SHIMOISHII
(C) CITY:OKAYAMA
(E) COUNTRY:JAPAN
(F) POSTAL CODE (ZIP):700

(v) COMPUTER READABLE FORM:
(A) MEDIUM TYPE:Floppy disk
(B) COMPUTER:IBM PC compatible
(C) OPERATING SYSTEM:PC-DOS/MS-DOS
(D) SOFTWARE:Word Perfect Version 5.0

(vii) PRIOR APPLICATION DATA:
(A1) APPLICATION NUMBER:JP 242137/94
(B1) FILING DATE:September 10, 1994
(A2) APPLICATION NUMBER:JP 200204/95
(B2) FILING DATE:July 14, 1995
(A3) APPLICATION NUMBER:JP 200221/95
(B3) FILING DATE:July 14, 1995

(2)INFORMATION FOR SEQ ID NO:1:
(i)SEQUENCE CHARACTERISTICS:
(A)LENGTH:11 amino acids
(B)TYPE:amino acid
(D)TOPOLOGY:linear
(ii)MOLECULE TYPE:peptide
(xi)SEQUENCE DESCRIPTION:SEQ ID NO:1:

Lys Val Asp Gly Ile Ile Ala Ala Tyr Gln Asn
1               5                   10

(3)INFORMATION FOR SEQ ID NO:2:
(i)SEQUENCE CHARACTERISTICS:
(A)LENGTH:10 amino acids
(B)TYPE:amino acid
(D)TOPOLOGY:linear
(ii)MOLECULE TYPE:peptide
(xi)SEQUENCE DESCRIPTION:SEQ ID NO:2:

Ile Ile Ala Ala Tyr Gln Asn Pro Ala Ser
1               5                   10

(4) INFORMATION FOR SEQ ID NO:3:
(i) SEQUENCE CHARACTERISTICS:
(A) LENGTH:10 amino acids
(B) TYPE:amino acid
(D) TOPOLOGY:linear
(ii) MOLECULE TYPE:peptide
(xi) SEQUENCE DESCRIPTION:SEQ ID NO:3:

Asn Arg Ile Trp Leu Gln Phe Ala Lys Leu
1               5                   10

(5) INFORMATION FOR SEQ ID NO:4:
(i) SEQUENCE CHARACTERISTICS:
(A) LENGTH:10 amino acids
(B) TYPE:amino acid
(D) TOPOLOGY:linear
(ii) MOLECULE TYPE:peptide
(xi) SEQUENCE DESCRIPTION:SEQ ID NO:4:

Phe Ala Ser Lys Asn Phe His Leu Gln Lys
1               5                   10

(6) INFORMATION FOR SEQ ID NO:5:
(i) SEQUENCE CHARACTERISTICS:
(A) LENGTH:11 amino acids
(B) TYPE:amino acid
(D) TOPOLOGY:linear
(ii) MOLECULE TYPE:peptide
(xi) SEQUENCE DESCRIPTION:SEQ ID NO:5:

Ser Leu Lys Leu Thr Ser Gly Lys Ile Ala Ser
1               5                   10

(7) INFORMATION FOR SEQ ID NO:6:
(i) SEQUENCE CHARACTERISTICS:
(A) LENGTH:8 amino acids
(B) TYPE:amino acid
(D) TOPOLOGY:linear
(ii) MOLECULE TYPE:peptide
(xi) SEQUENCE DESCRIPTION:SEQ ID NO:6:

Leu Thr Leu Arg Thr Ala Thr Asn
1               5

(8) INFORMATION FOR SEQ ID NO:7:
(i) SEQUENCE CHARACTERISTICS:
(A) LENGTH:5 amino acids
(B) TYPE:amino acid
(D) TOPOLOGY:linear
(ii) MOLECULE TYPE:peptide
(xi) SEQUENCE DESCRIPTION:SEQ ID NO:7:

Ala Phe Asn Val Glu
1               5

(9) INFORMATION FOR SEQ ID NO:8:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:8:

Lys Val Asp Gly Ile Ile Ala Ala Tyr Gln Asn Pro Ala Ser Trp Lys
1               5                   10                  15
Asn

(10) INFORMATION FOR SEQ ID NO:9:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:9:

Asn Arg Ile Trp Leu Gln Phe Ala Lys Leu Thr Gly Phe Thr Leu Met
1               5                   10                  15
Gly

(11) INFORMATION FOR SEQ ID NO:10:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:10:

Asp Ile Phe Ala Ser Lys Asn Phe His Leu Gln Lys Asn Thr Ile Gly
1               5                   10                  15
Thr

(12) INFORMATION FOR SEQ ID NO:11:
(i) SEQUENCE CHARACTERISTICS:

(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:11:

Asp Ile Ser Leu Lys Leu Thr Ser Gly Lys Ile Ala Ser Cys Leu Asn
1               5                   10                  15
Asp

(13) INFORMATION FOR SEQ ID NO:12:
(i) SEQUENCE CHARACTERISTICS:
(A) LENGTH:14 amino acids
(B) TYPE:amino acid
(D) TOPOLOGY:linear
(ii) MOLECULE TYPE:peptide
(xi) SEQUENCE DESCRIPTION:SEQ ID NO:12:

Leu Thr Leu Arg Thr Ala Thr Asn Ile Trp Ile Asp His Asn
1               5                   10

(14) INFORMATION FOR SEQ ID NO:13:
(i) SEQUENCE CHARACTERISTICS:
(A) LENGTH:14 amino acids
(B) TYPE:amino acid
(D) TOPOLOGY:linear
(ii) MOLECULE TYPE:peptide
(xi) SEQUENCE DESCRIPTION:SEQ ID NO:13:

Gly Gly Asn Ile Tyr Thr Lys Lys Glu Ala Phe Asn Val Glu
1               5                   10

(15) INFORMATION FOR SEQ ID NO:14:
(i) SEQUENCE CHARACTERISTICS:
(A) LENGTH:514 amino acids
(B) TYPE:amino acid
(D) TOPOLOGY:linear
(ii) MOLECULE TYPE:protein
(vi) ORIGINAL SOURCE:
(A) ORGANISM:Cryptomeria japonica
(F) TISSUE TYPE:pollen
(xi) SEQUENCE DESCRIPTION:SEQ ID NO:14:

Met Ala Met Lys Phe Ile Ala Pro Met Ala Phe Val Ala Met Gln Leu
1               5                   10                  15
Ile Ile Met Ala Ala Ala Glu Asp Gln Ser Ala Gln Ile Met Leu Asp
            20                  25                  30
Ser Asp Ile Glu Gln Tyr Leu Arg Ser Asn Arg Ser Leu Arg Lys Val
        35                  40                  45
Glu His Ser Arg His Asp Ala Ile Asn Ile Phe Asn Val Glu Lys Tyr
    50                  55                  60

Gly Ala Val Gly Asp Gly Lys His Asp Cys Thr Glu Ala Phe Ser Thr
65                  70                  75                  80
Ala Trp Gln Ala Ala Cys Lys Lys Pro Ser Ala Met Leu Leu Val Pro
                85                  90                  95
Gly Asn Lys Lys Phe Val Val Asn Asn Leu Phe Phe Asn Gly Pro Cys
            100                 105                 110
Gln Pro His Phe Thr Phe Lys Val Asp Gly Ile Ile Ala Ala Tyr Gln
        115                 120                 125
Asn Pro Ala Ser Trp Lys Asn Asn Arg Ile Trp Leu Gln Phe Ala Lys
    130                 135                 140
Leu Thr Gly Phe Thr Leu Met Gly Lys Gly Val Ile Asp Gly Gln Gly
145                 150                 155                 160
Lys Gln Trp Trp Ala Gly Gln Cys Lys Trp Val Asn Gly Arg Glu Ile
                165                 170                 175
Cys Asn Asp Arg Asp Arg Pro Thr Ala Ile Lys Phe Asp Phe Ser Thr
            180                 185                 190
Gly Leu Ile Ile Gln Gly Leu Lys Leu Met Asn Ser Pro Glu Phe His
        195                 200                 205
Leu Val Phe Gly Asn Cys Glu Gly Val Lys Ile Ile Gly Ile Ser Ile
    210                 215                 220
Thr Ala Pro Arg Asp Ser Pro Asn Thr Asp Gly Ile Asp Ile Phe Ala
225                 230                 235                 240
Ser Lys Asn Phe His Leu Gln Lys Asn Thr Ile Gly Thr Gly Asp Asp
                245                 250                 255
Cys Val Ala Ile Gly Thr Gly Ser Ser Asn Ile Val Ile Glu Asp Leu
            260                 265                 270
Ile Cys Gly Pro Gly His Gly Ile Ser Ile Gly Ser Leu Gly Arg Glu
        275                 280                 285
Asn Ser Arg Ala Glu Val Ser Tyr Val His Val Asn Gly Ala Lys Phe
    290                 295                 300
Ile Asp Thr Gln Asn Gly Leu Arg Ile Lys Thr Trp Gln Gly Gly Ser
305                 310                 315                 320
Gly Met Ala Ser His Ile Ile Tyr Glu Asn Val Glu Met Ile Asn Ser
                325                 330                 335
Glu Asn Pro Ile Leu Ile Asn Gln Phe Tyr Cys Thr Ser Ala Ser Ala
            340                 345                 350
Cys Gln Asn Gln Arg Ser Ala Val Gln Ile Gln Asp Val Thr Tyr Lys
        355                 360                 365
Asn Ile Arg Gly Thr Ser Ala Thr Ala Ala Ala Ile Gln Leu Lys Cys
    370                 375                 380
Ser Asp Ser Met Pro Cys Lys Asp Ile Lys Leu Ser Asp Ile Ser Leu
385                 390                 395                 400
Lys Leu Thr Ser Gly Lys Ile Ala Ser Cys Leu Asn Asp Asn Ala Asn
                405                 410                 415
Gly Tyr Phe Ser Gly His Val Ile Pro Ala Cys Lys Asn Leu Ser Pro
            420                 425                 430
Ser Ala Lys Arg Lys Glu Ser Lys Ser His Lys His Pro Lys Thr Val
        435                 440                 445
Met Val Lys Asn Met Gly Ala Tyr Asp Lys Gly Asn Arg Thr Arg Ile
    450                 455                 460
Leu Leu Gly Ser Arg Pro Pro Asn Cys Thr Asn Lys Cys His Gly Cys
465                 470                 475                 480
Ser Pro Cys Lys Ala Lys Leu Val Ile Val His Arg Ile Met Pro Gln
                485                 490                 495
Glu Tyr Tyr Pro Gln Arg Trp Met Cys Ser Arg His Ala Lys Ile Tyr
            500                 505                 510
His Pro

(16) INFORMATION FOR SEQ ID NO:15:
(i) SEQUENCE CHARACTERISTICS:
(A) LENGTH:353 amino acids
(B) TYPE:amino acid
(D) TOPOLOGY:linear
(ii) MOLECULE TYPE:protein
(vi) ORIGINAL SOURCE:
(A) ORGANISM:Cryptomeria japonica
(F) TISSUE TYPE:pollen
(xi) SEQUENCE DESCRIPTION:SEQ ID NO:15:

Asp Asn Pro Ile Asp Ser Cys Trp Arg Gly Asp Ser Asn Trp Ala Gln
1               5                   10                  15
Asn Arg Met Lys Leu Ala Asp Cys Ala Val Gly Phe Gly Ser Ser Thr
            20                  25                  30
Met Gly Gly Lys Gly Gly Asp Leu Tyr Thr Val Thr Asn Ser Asp Asp
        35                  40                  45
Asp Pro Val Asn Pro Ala Pro Gly Thr Leu Arg Tyr Gly Ala Thr Arg
    50                  55                  60
Asp Arg Pro Leu Trp Ile Ile Phe Ser Gly Asn Met Asn Ile Lys Leu
65                  70                  75                  80
Lys Met Pro Met Tyr Ile Ala Gly Tyr Lys Thr Phe Asp Gly Arg Gly
                85                  90                  95
Ala Gln Val Tyr Ile Gly Asn Gly Gly Pro Cys Val Phe Ile Lys Arg
            100                 105                 110
Val Ser Asn Val Ile Ile His Gly Leu Tyr Leu Tyr Gly Cys Ser Thr
        115                 120                 125
Ser Val Leu Gly Asn Val Leu Ile Asn Glu Ser Phe Gly Val Glu Pro
    130                 135                 140
Val His Pro Gln Asp Gly Asp Ala Leu Thr Leu Arg Thr Ala Thr Asn
145                 150                 155                 160
Ile Trp Ile Asp His Asn Ser Phe Ser Asn Ser Ser Asp Gly Leu Val
                165                 170                 175
Asp Val Thr Leu Thr Ser Thr Gly Val Thr Ile Ser Asn Asn Leu Phe
            180                 185                 190
Phe Asn His His Lys Val Met Leu Leu Gly His Asp Asp Ala Tyr Ser
        195                 200                 205
Asp Asp Lys Ser Met Lys Val Thr Val Ala Phe Asn Gln Phe Gly Pro
    210                 215                 220
Asn Cys Gly Gln Arg Met Pro Arg Ala Arg Tyr Gly Leu Val His Val
225                 230                 235                 240
Ala Asn Asn Asn Tyr Asp Pro Trp Thr Ile Tyr Ala Ile Gly Gly Ser
                245                 250                 255
Ser Asn Pro Thr Ile Leu Ser Glu Gly Asn Ser Phe Thr Ala Pro Asn
            260                 265                 270
Glu Ser Tyr Lys Lys Gln Val Thr Ile Arg Ile Gly Cys Lys Thr Ser
        275                 280                 285
Ser Ser Cys Ser Asn Trp Val Trp Gln Ser Thr Gln Asp Val Phe Tyr
    290                 295                 300
Asn Gly Ala Tyr Phe Val Ser Ser Gly Lys Tyr Glu Gly Gly Asn Ile
305                 310                 315                 320
Tyr Thr Lys Lys Glu Ala Phe Asn Val Glu Asn Gly Asn Ala Thr Pro
                325                 330                 335
Gln Leu Thr Lys Asn Ala Gly Val Leu Thr Cys Ser Leu Ser Lys Arg

            340                 345                 350
Cys

(17) INFORMATION FOR SEQ ID NO:16:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:16:

Asn Arg Ile Trp Leu Gln Phe Ala Lys Leu Gln Gly Phe Thr Leu Met
1               5                   10                  15
Gly

(18) INFORMATION FOR SEQ ID NO:17:
(i) SEQUENCE CHARACTERISTICS:
(A) LENGTH:14 amino acids
(B) TYPE:amino acid
(D) TOPOLOGY:linear
(ii) MOLECULE TYPE:peptide
(xi) SEQUENCE DESCRIPTION:SEQ ID NO:17:

Leu Ala Leu Arg Thr Ala Thr Asn Ile Trp Ile Asp His Asn
1               5                   10

(19) INFORMATION FOR SEQ ID NO:18:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:18:

Lys Val Asp Gly Ile Ile Ala Ala Tyr Gln Asn Ala Ala Ala Ala Ala
1               5                   10                  15
Ala

(20) INFORMATION FOR SEQ ID NO:19:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:19:

Ala Ala Ala Ala Ile Ile Ala Ala Tyr Gln Asn Pro Ala Ser Ala Ala
1               5                   10                  15
Ala

(21) INFORMATION FOR SEQ ID NO:20:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:20:

Asn Arg Ile Trp Leu Gln Phe Ala Lys Leu Ala Ala Ala Ala Ala Ala
1               5                   10                  15
Ala

(22) INFORMATION FOR SEQ ID NO:21:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:21:

Ala Ala Phe Ala Ser Lys Asn Phe His Leu Gln Lys Ala Ala Ala Ala
1               5                   10                  15
Ala

(23) INFORMATION FOR SEQ ID NO:22:
(i) SEQUENCE CHARACTERISTICS:
(A) LENGTH:17 amino acids
(B) TYPE:amino acid
(D) TOPOLOGY:linear
(ii) MOLECULE TYPE:peptide
(xi) SEQUENCE DESCRIPTION:SEQ ID NO:22:

Ala Ala Ser Leu Lys Leu Thr Ser Gly Lys Ile Ala Ser Ala Ala Ala
1               5                   10                  15
Ala

(24) INFORMATION FOR SEQ ID NO:23:
(i) SEQUENCE CHARACTERISTICS:
(A) LENGTH:14 amino acids
(B) TYPE:amino acid
(D) TOPOLOGY:linear
(ii) MOLECULE TYPE:peptide
(xi) SEQUENCE DESCRIPTION:SEQ ID NO:23:

Leu Thr Leu Arg Thr Ala Thr Asn Ala Ala Ala Ala Ala Ala
1               5                   10

(25) INFORMATION FOR SEQ ID NO:24:
(i) SEQUENCE CHARACTERISTICS:
(A) LENGTH:14 amino acids
(B) TYPE:amino acid
(D) TOPOLOGY:linear
(ii) MOLECULE TYPE:peptide
(xi) SEQUENCE DESCRIPTION:SEQ ID NO:24:

Ala Ala Ala Ala Ala Ala Ala Ala Ala Ala Phe Asn Val Glu
1               5                   10

## Description

### Background of the Invention

### Field of the invention

The present invention relates to a novel peptide and uses of the same, more particularly, to a peptide which activates T-cells specific to cedar pollen allergens, and an immunotherapeutic agent containing the peptide as an effective ingredient.

### Description of the Prior Art

For the last tens and several years, the number of patients, who complain of rhinitis and conjunctivitis caused by cedar pollinosis, has been increasing year by year in Japan especially during spring. These symptoms have been received much publicity from the mass media and become a matter of concern which could not be ignored in view of the public health because the number of which is relatively large and because a variety of events and functions are continuously held.

Cedar pollinosis is a type of allergy, and the main causative is known to be an antigenic substance present in cedar pollens, i.e. a cedar pollen allergen. Invasion of such pollens scattered in the air into the body induces the formation of immunoglobulin E antibody specific to such pollens. When the body in such a state is re-invaded by the cedar pollens, cedar pollen allergens in the pollens immunologically react with immunoglobulin E antibody to cause an allergic symptom.

Now, it is known that cedar pollens contain at least 2 types of cedar pollen allergens with different antigenicities. The one is an allergen, now named "*Cry J* I" as reported by Yasueda et al. in *Journal of Allergy and Clinical Immunology*, Vol.71, No.1, pp.77-86 (1983), and the other is one, now named "*Cry j* II" as reported by Taniai et al. in *FEBS LETTERS*, Vol. 239, No.2, pp.329-332 (1988) and by Sakaguchi et al. in *Allergy*, Vol.45, pp.309-312 (1990). It is reported that cedar pollens generally contain *Cry j* I and *Cry j* II in a ratio of about 50:1 to 5:1, and almost all serum specimens collected from patients with cedar pollinosis react with these allergens. Sawatani et al. reported in "*Allergy*", Vol.42, No.6, pp.738-747 (1993) that *Cry j* II exerts the same level of antigenicity as *Cry j* I on the intradermal test or the radioallergosorbent test (RAST).

Several cedar pollen allergens were isolated and revealed their properties and features to some extent, and this may lead to some possibilities of treating and/or preventing cedar pollinosis by administering purified cedar pollen allergens to human to hyposensitize. Recently, some hyposensitization agents for such purposes were proposed, for example, in Japanese Patent Laid-Open Nos.156,926/89 and 93,730/91 which disclose a method to administer to human a conjugate as a hyposensitization agent prepared by covalently bonding a saccharide with a cedar pollen allergen represented by an amino acid sequence containing the N-terminal of Asp-Asn-Pro-Ile-Asp-Ser or Ala-Ile-Asn-Ile-Phe-Asn. However, the diagnosis of allergic diseases and hyposensitization treatment generally require a relatively large amount of highly purified allergens, and cedar pollens only contain a relatively small amount of cedar pollen allergens and have a relatively poor stability, and such activities would accompany many difficulties when the pollens should be used alone as a diagnostic- or hyposensitization-agent.

In view of the foregoing, unlike conventional ones which administer intact allergens to patients, the latest treatment and prevention of allergic diseases are focused on immunotherapies which administer to patients a substance, having a relatively narrow region of an allergen, which is specifically recognized by T-cells, i.e. a relatively-low molecular weight peptide consisting essentially of T-cell epitope(s).

In general, allergens are first digested when captured by antigen presenting cells such as macrophage, then the digested fragments link to HLA proteins on the surface of the antigen presenting cells and induce antigenicity. The antigen presenting fragments are restricted to specific regions of particular sites of allergens due to their affinity to the HLA proteins. Among these regions, those which are specifically recognized by T-cells are generally called "T-cell epitopes". Immunotherapy to administer to patients a peptide which essentially consists of T-cell epitope(s) has the following advantage:
(i) Such a peptide lacks B-cell epitopes, meaning that it does not react with immunoglobulin E antibody specific to the allergen, and that side effects such as anaphylaxis frequently caused by conventional crude- and purified-allergens, would not occur; and
(2) As compared with conventional hyposensitization agents, such an immunotherapy can shorten by a large margin the therapeutic period of time required for reaching to an effective dose level from the initiation of a relatively-low dose level.

Although T-cell epitopes of various allergens have been analyzed energetically, these analyses generally require the total amino acid sequences of the allergens. So far as cedar pollen allergens are concerned, their T-cell epitopes have not actually been analyzed.

### Summary of the Invention

In view of the foregoing, the first object of the present invention is to provide a peptide essentially consisting of T-cell epitope(s) of cedar pollen allergens, and homologous peptides thereunto.

The second object of the present invention is to provide an immunotherapeutic agent which contains the peptide as an effective ingredient.

The first object of the present invention is attained by a peptide which does not substantially react with immunoglobulin E antibody specific to cedar pollen allergens, and significantly more activates T-cells specific to the cedar pollen allergens than a negative control when assayed by the ³H-thymidine uptake method.

The second object of the present invention is attained by an immunotherapeutic agent which contains the peptide as an effective ingredient.

### Detailed Description of the Invention

The peptide according to the present invention does not react with immunoglobulin E antibody specific to cedar pollen allergens, and activates T-cells specific to the allergens without substantially inducing anaphylaxis when administered to mammals in general including human.

The immunotherapeutic agent containing the peptide according to the present invention exerts a relatively high therapeutic- and/or prophylactic-effects on cedar pollinosis without substantially inducing anaphylaxis when administered to mammals in general including human.

Explaining now the present invention with reference to the following Experiments and Examples, the present invention is accomplished based on the finding of a peptide which essentially consists of T-cell epitope(s) of cedar pollen allergens.

As a result of the long term research, the present inventors found last year that cedar pollen allergens have the amino acid sequence in SEQ ID NO:14 as a main ingredient, and disclosed the finding in Japanese Patent Application No.344,596/93. International Patent Application Laid-Open No.93/01,213 discloses that an another component of cedar pollen allergens has the amino acid sequence in SEQ ID NO:15, and the present inventors also reported the same amino acid sequence in *The 6th Symposium of Japanese Society of Allergology*, held in April 14 to 16, 1994, in Kumamoto-city, Kumamoto, Japan.

To reveal T-cell epitopes of cedar pollen allergens, the present inventors synthesized about 180 peptides, which consist of 11, 14 or 17 successive amino acid residues in the amino acid sequences in SEQ ID NOs:14 and 15 and have different amino acid sequences, based on the amino acid sequences in SEQ ID NOs:14 and 15, and tested for reactivity with immunoglobulin E antibody specific to the allergens, as well as for function of activating T-cells specific to the allergens. As a result, it was found that the peptides having the amino acid sequences in SEQ ID NOs:8 to 13 do not substantially react with immunoglobulin E antibody specific to cedar pollen allergens, and, as compared with a negative control, significantly activate T-cells specific to the allergens when assayed by the ³H-thymidine uptake method. These facts indicate that the peptides essentially consist of T-cell epitopes of cedar pollen allergens. The analysis of these amino acid sequences in SEQ ID NOs:8 to 13 revealed that those in SEQ ID NOs:1 to 7 are essential for T-cells to recognize peptides having the amino acid sequences in SEQ ID NOs:8 to 13.

The following Experiments 1 and 2 explain how revealed these facts were:

### Experiment 1

### Preparation of peptide and cedar pollen allergen

### Experiment 1-1

### Preparation of peptide

As is described above, it is known that cedar pollens contain at least 2 types of allergens with different properties and features. The mature proteins of these cedar pollen allergens are revealed to have the amino acid sequence in SEQ ID NO:14 or 15, and, actually, a cedar pollen allergen having an amino acid sequence corresponding to that positioning at 46 to 433 amino acids or that positioning at 51 to 433 amino acids in SEQ ID NO:14 (hereinafter designated as "allergen A"), and a cedar pollen allergen having an amino acid sequence corresponding to that positioning at 1 to 353 amino acids in SEQ ID NO:15 (hereinafter designated as "allergen B") were isolated from cedar pollens. In SEQ ID NO:14, the initial amino acid residue in the N-terminal amino acid sequence, which was decoded with a base sequence of a cDNA, is provisionally marked "1" because no signal peptide of the gene coding for allergen A has been revealed.

Ninety-five peptides (samples A-1 to A-95) with different amino acid sequences consisting of 11 or 14 amino acid residues were chemically synthesized in this experiment by duplicating amino acid residues 10 by 10 over the region of the amino acids positioning at 46 to 433 in SEQ ID NO:14, while 86 peptides (samples B-1 to B-86) with different amino acid sequences which consist of 14 amino acid residues were chemically synthesized by duplicating amino acid residues 10 by 10 over the region of the amino acids positioning at 1 to 353 in SEQ ID NO:15. These peptides were used for screening the present polypeptide in the following Experiment 2.

One hundred and eighty-one peptides, which consist of 11 or 14 amino acid residues and contain either of the amino acid sequences in SEQ ID NOs:1 to 6, were synthesized with "CLEAVABLE PEPTIDE KIT" commercialized by Cambridge Research Biochemicals, Cheshire, United Kingdom, and sampled for analysis on "MODEL 470 A", a peptide sequencer commercialized by Perkin-Elmer Corp., Instrument Div., Norrwalk, USA, and revealed that they had prescribed amino acid sequences.

### Experiment 1-2

### Preparation of cedar pollen allergen

One part by weight of a cedar pollen collected from male flower of *Ura-sugi*, a Japanese cedar (*Cryptomeria japonica*) grown in Akita, Japan, was soaked in about 16 parts by weight of 0.125 M aqueous sodium bicarbonate solution (pH 8.2) and extracted at 4°C for one hour under gentle stirring conditions. The resultant extract was centrifuged, and the precipitate was re-extracted similarly as above, followed by pooling the first-and second-supernatants. The pooled solution was mixed with cetavlon to give a concentration of 0.1 w/v %, and allowed to stand at 4°C for one hour under gentle stirring conditions to precipitate polysaccharides. The resultant mixture was centrifuged, and the resultant supernatant was mixed with ammonium sulfate to give a saturation degree of 80 w/v %, and salted out by standing at 4°C overnight.

The precipitate was collected and dialyzed against 50 mM Tris-HCl buffer (pH 7.8) for 10 hours, and the resultant dialyzed solution was filtered, fed to a column of DEAE-SEPHADEX which had been equilibrated with 50 mM Tris-HCl buffer (pH 7.8), and fed with a fresh preparation of the same buffer to elute fractions containing proteinaceous components. The fractions were pooled, adjusted to pH 5.0 by the addition of acetic acid, and fed to a column of CM-SEPHADEX which had been equilibrated with 10 mM acetate buffer (pH 5.0). The column was washed with 10 mM acetate buffer (pH 5.0) and fed with 0.1 M phosphate buffer (pH 7.0) containing 0.3 M sodium chloride, followed by collecting and pooling fractions containing proteinaceous components.

The solution thus obtained was fed to a column of "MONO S" which had been previously equilibrated with 10 mM acetate buffer (pH 5.0), and the column was washed with 10 mM acetate buffer (pH 5.0), fed with a linear gradient buffer ranging from 0 M to 0.5 M sodium chloride in 10 mM phosphate buffer (pH 7.0), followed by eluting from the column allergen B and allergen A at around 0.1-0.3 M sodium chloride and at around 0.4 M sodium chloride, respectively. Fractions containing allergens A and B were separatory collected, concentrated into a desired level, and lyophilized for use in Experiment 2. The yields of allergens A and B were respectively about 0.01% and 0.02% to the material cedar pollen, on a dry solid basis.

### Experiment 2

### Screening of peptide containing T-cell epitope of cedar pollen allergen

### Experiment 2-1

### Activation of T-cell specific to cedar pollen allergen

A heparinized peripheral blood, prepared with a patient with cedar pollinosis, was subjected to the ficoll-hypaque gradient centrifugation to isolate a mononuclear cell population containing T-cells specific to cedar pollen allergens. The population was suspended in RPMI 1640 medium (pH 7.0) supplemented with 5 v/v % AB serum, and the suspension was distributed to 96-well microplates to give 5x10⁵ cells/well. One µg/well of the peptide in Experiment 1-1 or the cedar pollen allergen in Experiment 1-2 was added to the microplates, and each well in the microplates was volumed up to 200 µl/well with a fresh preparation of the same medium, followed by the incubation of the microplates in an incubator at 37°C for 2 days under 5 v/v % CO₂ conditions. Thereafter, ³H-thymidine was added to the microplates in an amount of 1.0 µCi/well, followed by the incubation for 16 hours under the same conditions and the assay for the content of ³H-thymidine uptake in the mononuclear cell population by conventional method using a scintillation counter. As a control, a system free of the peptide and the cedar pollen allergen was provided and treated similarly as above.

The activating activity to T-cells specific to cedar pollen allergens was judged based on the level of ³H-thymidine uptake (cpm) in the mononuclear cell population containing the same T-cells. When the systems which did or did not exceed the uptake level by about 2-fold or higher of that of the control system, they were respectively marked "positive" and "negative". The results were in Tables 1 to 6.

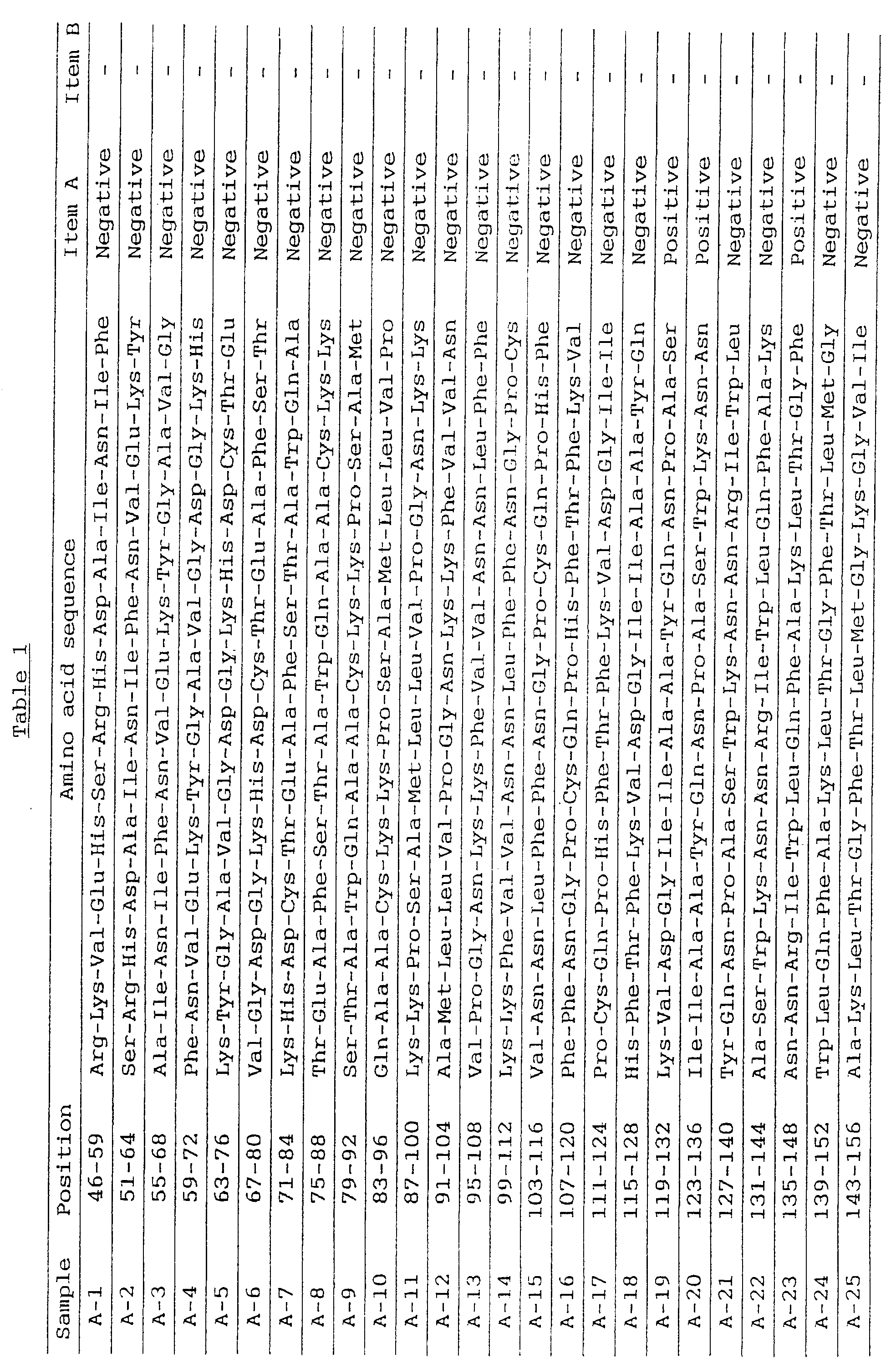

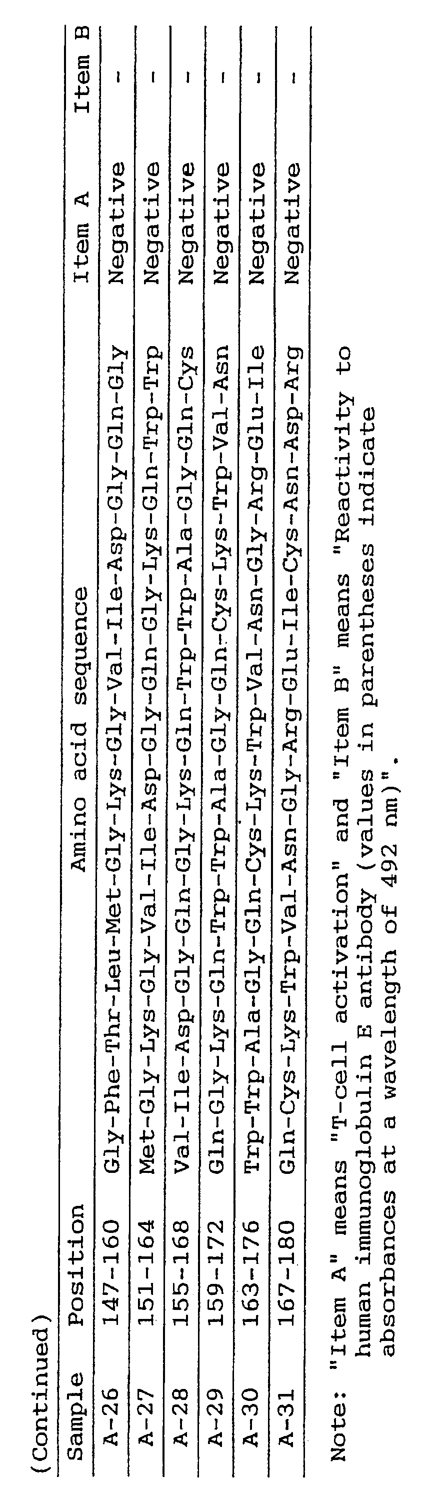

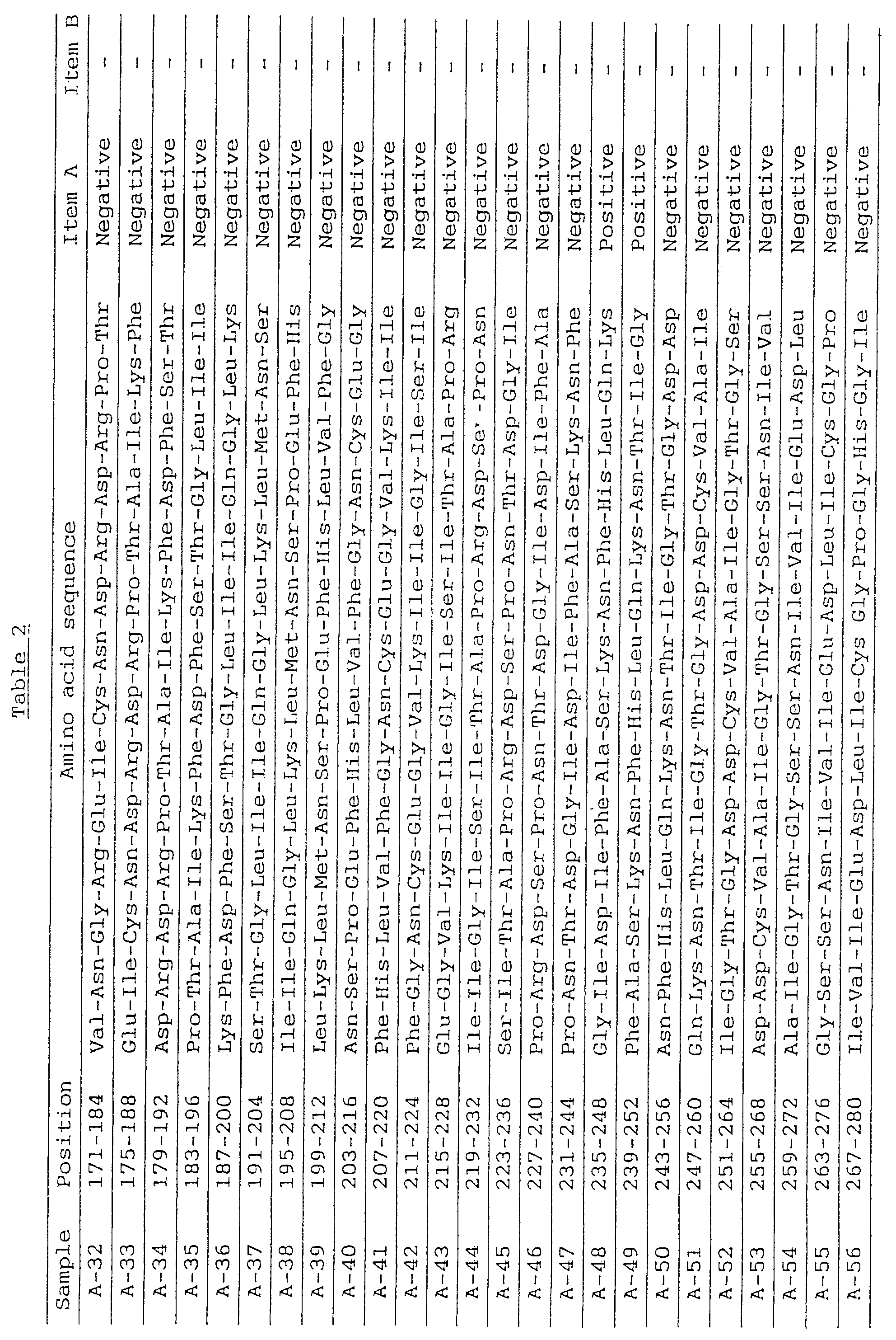

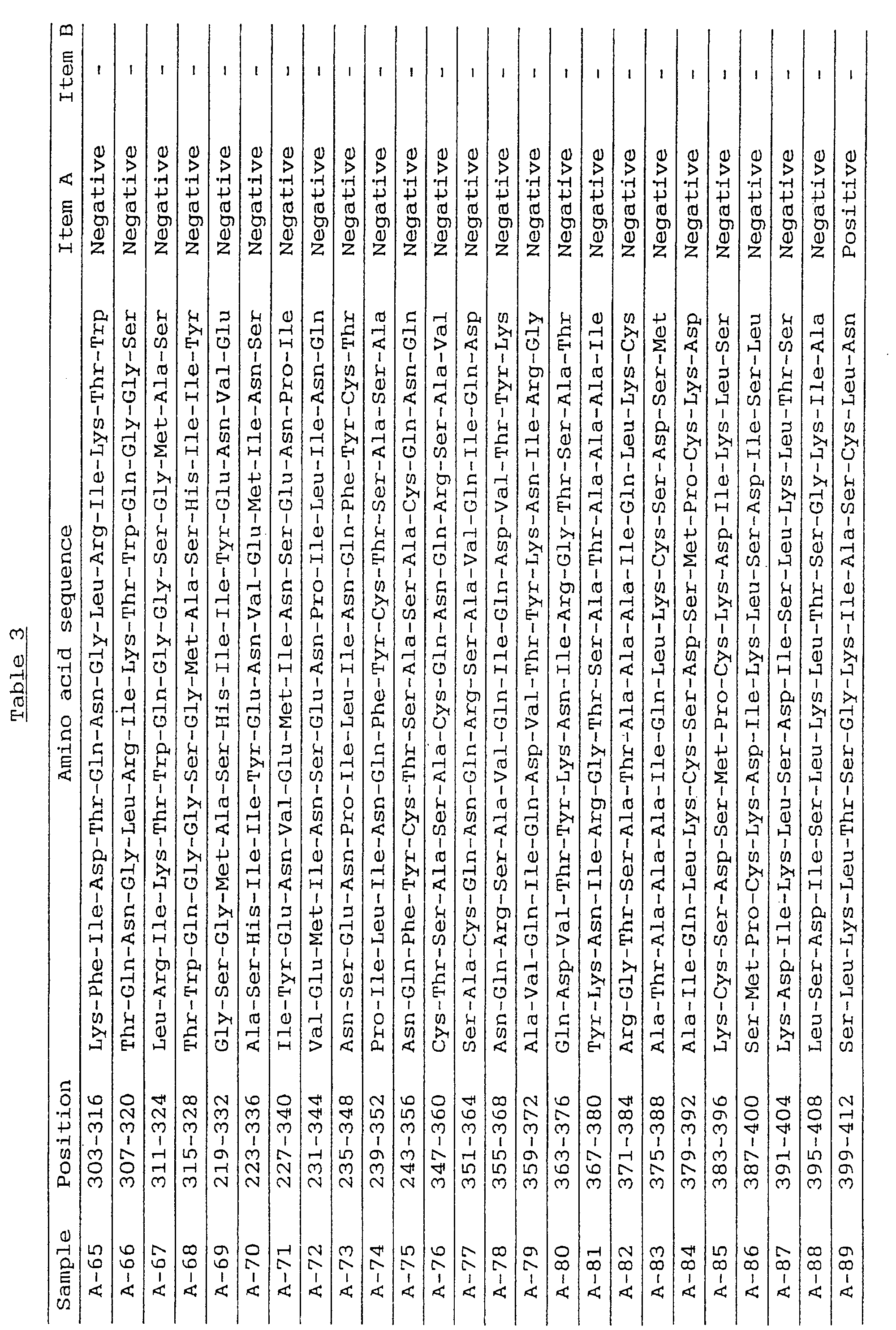

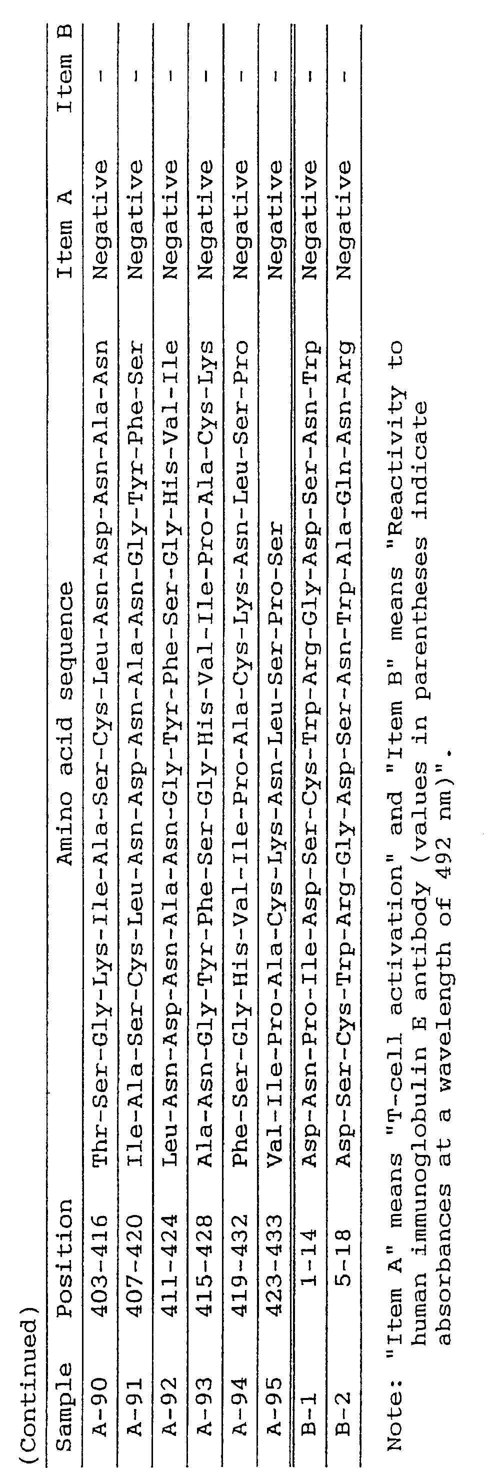

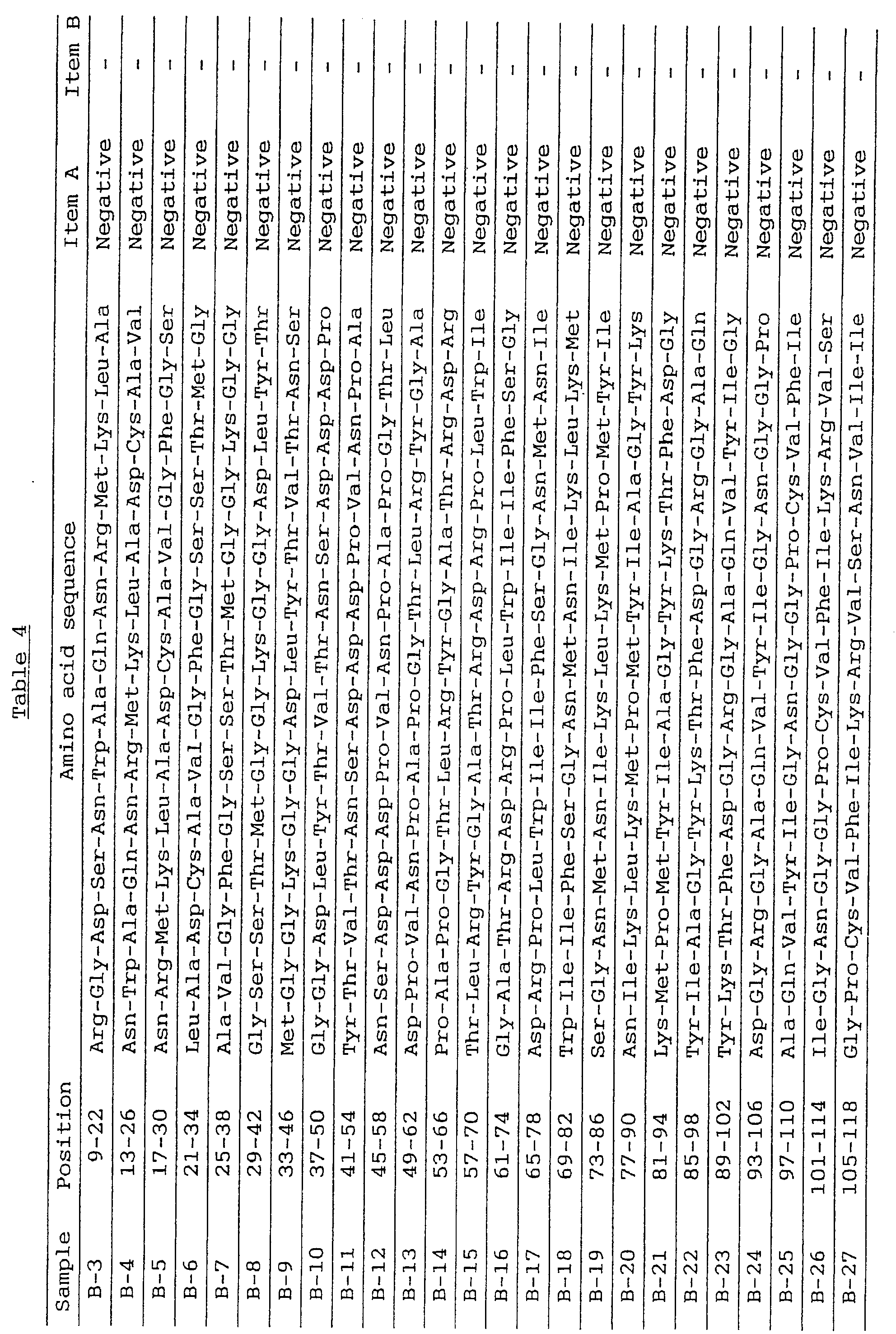

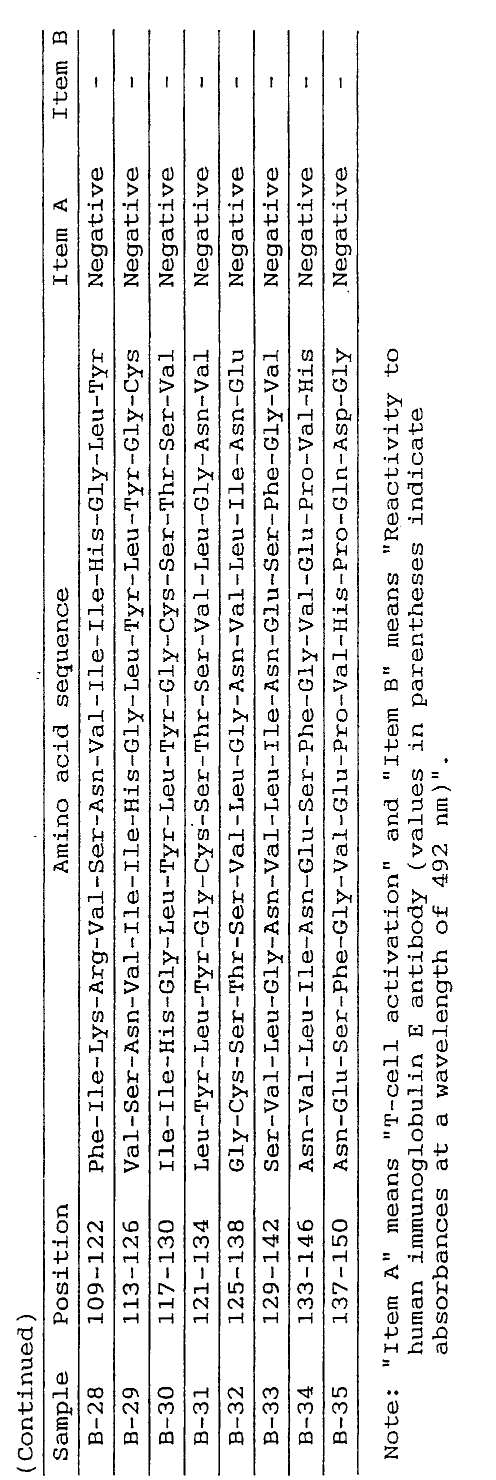

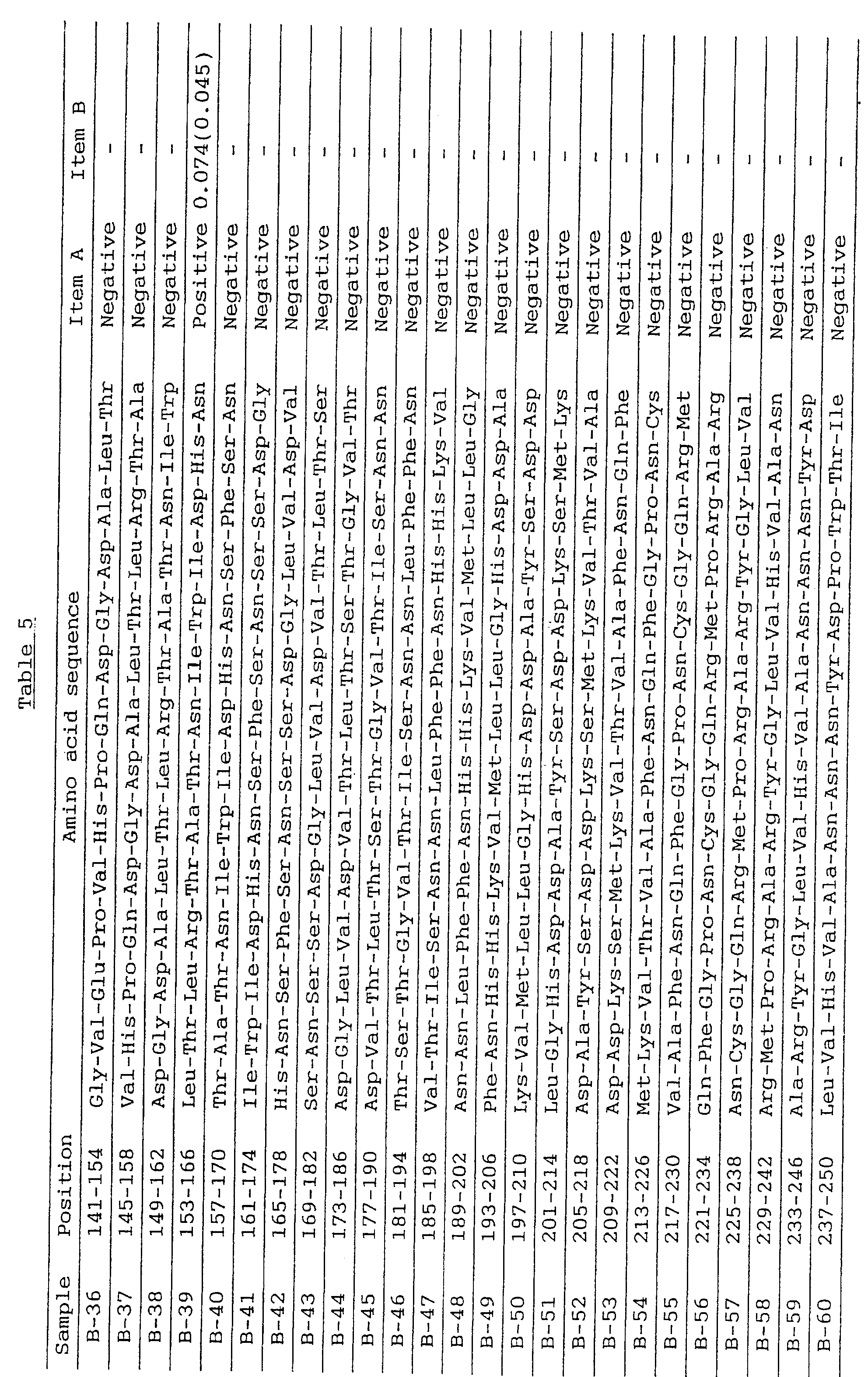

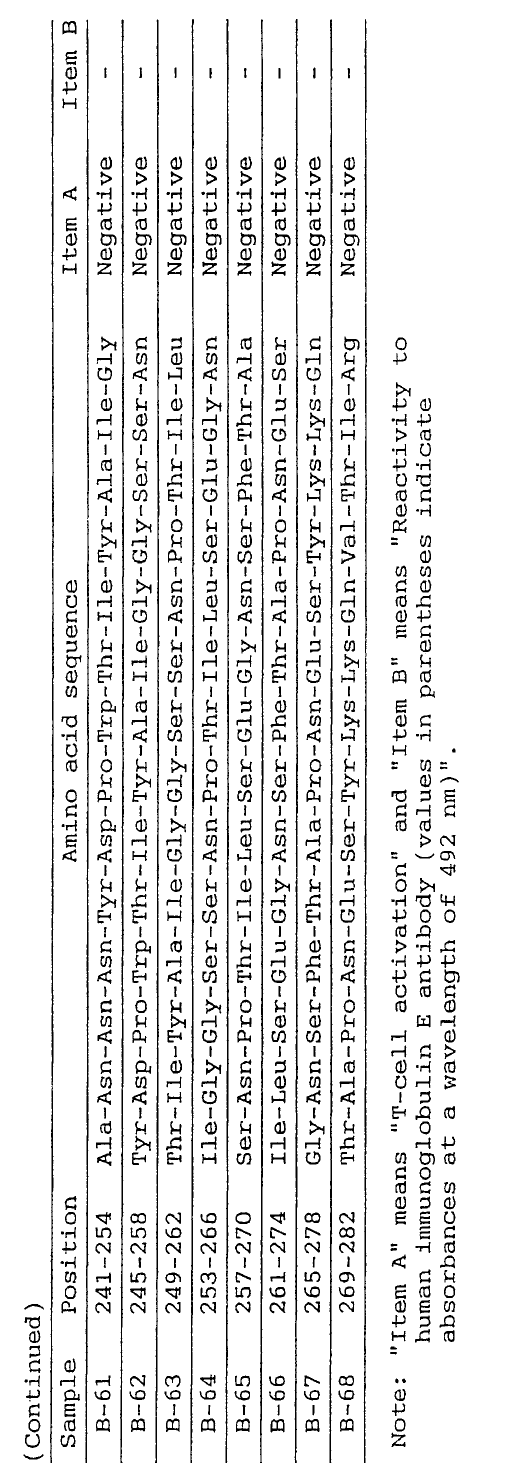

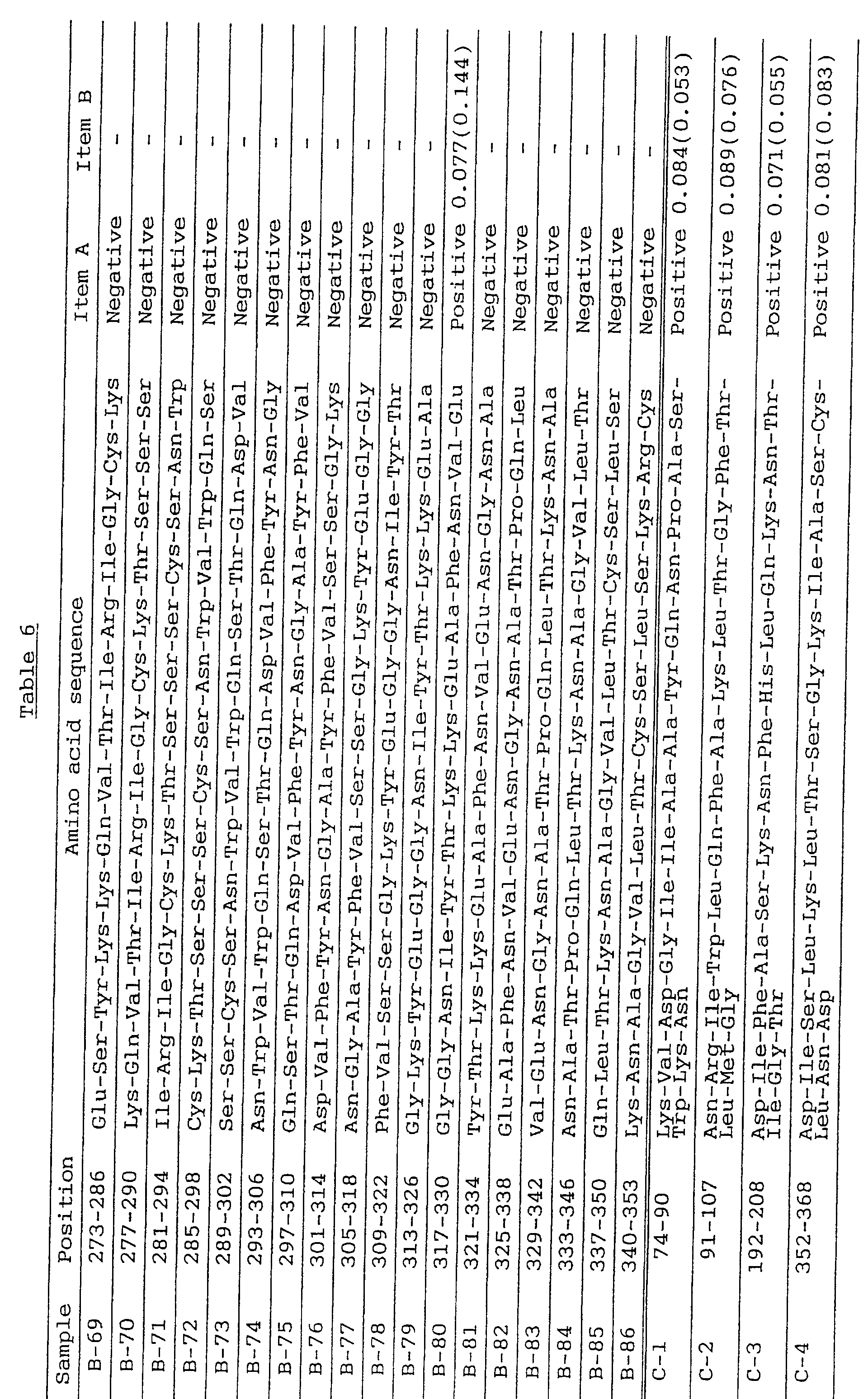

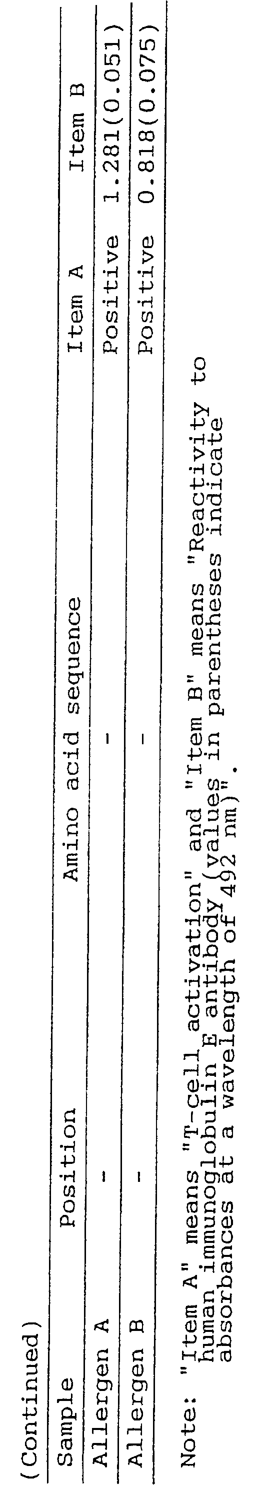

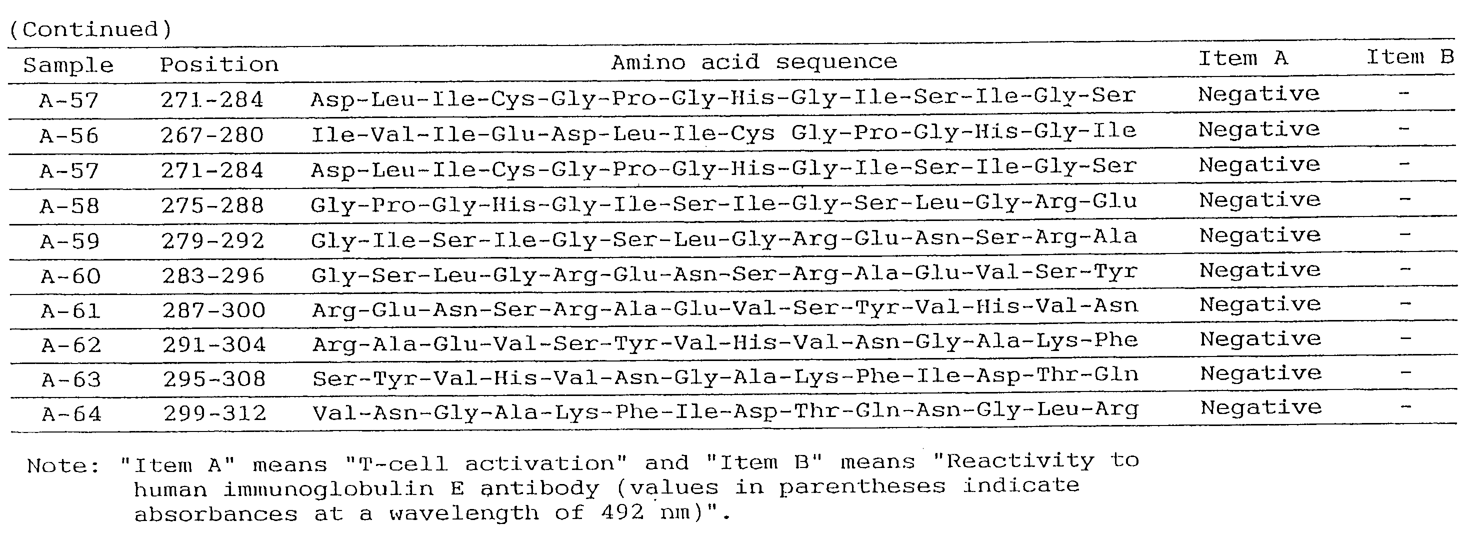

The results in Tables 1 to 6 indicate that the peptide and the cedar pollen allergen used in the above experiments clearly acted differently on T-cells specific to the allergen. The system with the sample A-19, A-20, A-23, A-48, A-49, A-89, B-39 or B-80, or the allergen A or B significantly enhanced the ³H-thymidine uptake as compared with a negative control, while systems with other samples did not significantly increase the uptake. These results show that the samples A-19, A-20, A-23, A-48, A-49, A-89, B-39 and B-80, and the allergens A and B significantly activated the T-cells in mononuclear cell population specific to cedar pollen allergens

With respect to the samples A-19, A-20, A-48 and A-49 which had common and duplicated amino acid sequences, and to the samples A-23 and A-89 which showed a slightly lower T-cell activating activity than those of other T-cell activating activity positive samples, peptides, which have amino acid sequences of SEQ ID NOs:8 to 11 and consist of 17 amino acid residues, were chemically synthesized.

According to conventional methods, peptides, i.e. the samples C-1 to C-4 having the amino acid sequences in SEQ ID NOs:8 to 11, were synthesized by using "EXCELL®", a peptide synthesizer commercialized by Milligen/Biosearch, Division of Millipore, Massachusetts, USA, and purified up to give a purity of 95 w/w %, on a dry solid basis (d.s.b.), on reverse phase high-performance liquid chromatography using "MODEL Hi-Pore RP-318", a column commercialized by Bio-Rad Laboratories Inc., Brussels, Belgium. Thereafter, the samples C-1 to C-4 were sampled for analysis on "MODEL 470A", a peptide sequencer commercialized by Perkin-Elmer Corp., Instrument Div., Norrwalk, USA, and revealed that all the samples had the prescribed amino acid sequences.

Similarly as above, the samples C-1 to C-4 were tested for T-cell activating activity and revealed to be positive, meaning that they significantly activate T-cells specific to cedar pollen allergens.

### Experiment 2-2

### Reactivity to immunoglobulin E antibody specific to cedar pollen allergen

By using EIA (enzyme immunoassay) as reported by Taniai et al. in "*Molecular Immunology*", Vol.30, No.2, pp.183-189 (1993), the samples B-39, B-80, C-1 and C-4, and the allergens A and B, which had been revealed in Experiment 2-1 to significantly activate T-cells speciflc to cedar pollen allergens, were studied on reactivity with immunoglobulin E antibody specific to cedar pollen allergens prepared from blood of a patient with cedar pollinosis.

One g of bis(sulfosuccinimidyl)suberate (BS₃) purchased from Pierce, Rockford, Illinois, USA, was dissolved in 10 ml distilled water, and 50 µl/well of the solution was distributed to "COVALINK MODULE", a 96-well microplate commercialized by Nunc, Roskilde, Denmark, followed by incubating the microplate at 37°C for 3 hours. The microplate was washed with distilled water, injected with 50 µl/well of a solution prepared by dissolving in phosphate buffered saline (PBS) 20 µg/ml or 5 µg/ml of any one of the samples B-39, B-80, C-1 to C-4, and the allergens A and B, followed by incubating the microplate at 37°C for 3 hours to covalently bond the samples and the allergens on the surfaces of the microplate. Fifty µl/well of PBS containing one w/v % calf serum albumin was added to the microplate which was then allowed to stand at 4°C overnight to block intact active groups of the samples and the allergens, and washed with PBS containing 0.1 w/v % calf serum albumin, followed by adding to the microplate 50 µl/well of serum of a patient with cedar pollinosis which had been diluted by 5 times with a fresh preparation of the same PBS with the same concentration of calf serum albumin, and incubating the microplate at 37°C for one hour.

The microplate was then washed with PBS containing 0.1 w/v % calf serum albumin, injected with 50 µl/well of a one µl/ml biotin-labelled anti-human ε chain antibody solution prepared by diluting the antibody in a fresh preparation of the same PBS with the same concentration of calf serum albumin, incubated at 37°C for one hour, washed with PBS containing 0.1 w/v % calf serum albumin, injected with 50 µl/well of a solution of peroxidase-labelled avidin, commercialized by Zymed, California, USA, prepared by diluting by 5,000 times with a fresh preparation of the same PBS with the same concentration of calf serum albumin, and further incubated at 37°C for one hour. The microplate was then washed with PBS containing 0.1 w/v % calf serum albumin, injected with 100 µl/well of 0.1 M citric acid/phosphate buffer (pH 5.0) containing 0.03 v/v % peroxide and 0.5 mg/ml of o-phenylenediamine, and allowed to stand at ambient temperature for 5 min to effect enzymatic reaction. One hundred µl/well of 2 N sulfuric acid was added to the microplate to suspend the enzymatic reaction, and measured for absorbance in usual manner with a spectrophotometer at a wavelength of 492 nm.

In parallel, a system as a negative control using serum of a healthy donor in place of the serum of the cedar pollinosis patient was provided, and treated similarly as above. The results were in Tables 1 to 6.

As is evident from the results in Tables 1 to 6, the allergens A and B strongly reacted with immunoglobulin E antibody specific to cedar pollen allergens prepared from patients with cedar pollinosis, but the samples B-39, B-80, and C-1 to C-4 did not substantially react with these allergens. This means that these samples are defective in B-cell epitopes of cedar pollen allergens contained in the allergens A and B. Considering systematically these results and the data in Experiment 2-1, it can be estimated that the above samples, i.e. the peptides, which have the amino acid sequences in SEQ ID NOs:8 to 13, essentially consist of T-cell epitopes of cedar pollen allergens.

### Experiment 3

### Screening for amino acid sequence essential for recognition of T-cell epitope by T-cell

In this experiment, the six T-cell epitopes revealed in Experiment 2 were further analyzed, and amino acid sequences essential for recognition by T-cells were screened.

According to the method in Experiment 1-1, peptides consisting of 14 or 17 amino acids were chemically synthesized by replacing with alanine one or more amino acids in either or both ends of the amino acid sequences in SEQ ID NOs:8 to 13 and those in the samples B-38, B-81 and B-82 which could not have be denied their T-cell activating activity. These peptides were studied on their activation of T-cells specific to cedar pollen allergens and their reactivity to immunoglobulin E antibody specific to the allergens.

As a result, as is shown in Table 7, it was found that the samples D-1 to D-7, containing the amino acid sequences in SEQ ID NOs:1 to 7, act on T-cells specific to cedar pollen allergens and immunoglobulin E antibody specific to the allergens almost similarly as the peptides having the amino acid sequences in SEQ ID NOs:8 to 13. These facts strongly indicate that the amino acid sequences in SEQ ID NOs:1 to 7 are essential for T-cells to recognize the peptides having the amino acid sequences in SEQ ID NOs:8 to 13.

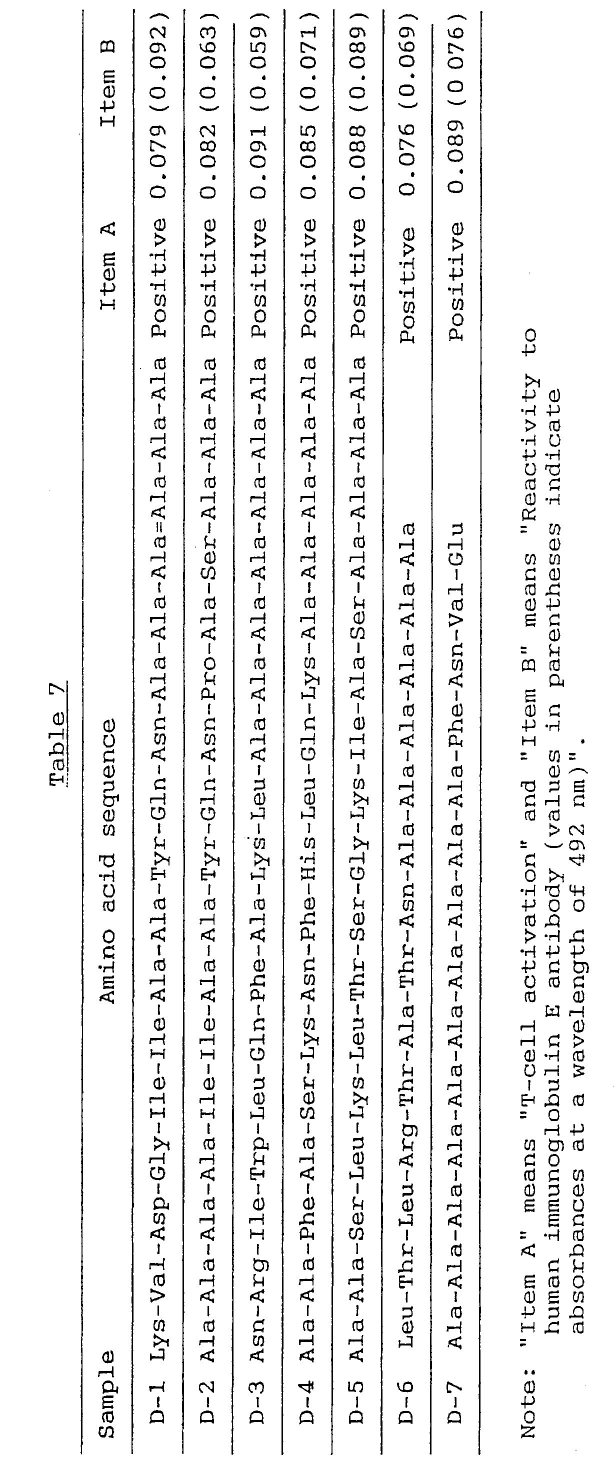

### Experiment 4

### Acute toxicity test

Immunotherapeutic agents obtained by the methods in Examples B-1 to B-7 were orally and peritoneally administered to mice, 20-day-old. As a result, it was found that the LD₅₀ of these agents is 200 mg/kg mouse or higher independently of their administration routes. This means that the peptide according to the present invention can be incorporated into immunotherapeutic agents for administering to mammals including human without fear of causing side effects.

As is described above, the present invention relates to a polypeptide which does not substantially react with immunoglobulin E antibody specific to cedar pollen allergens, and significantly more activates T-cells specific to the cedar pollen allergens than the negative control when assayed by the ³H-thymidine uptake method. The present invention includes any peptide as long as it has these properties independently of its structure, origin and preparation.

The peptide according to the present invention usually consists of 5-50 amino acids, preferably, 10-20 amino acids which link each other via the peptide bonding. Examples of such a peptide include those having the amino acid sequences in SEQ ID NOs:8 to 13, and homologous ones thereunto. These homologous amino acid sequences can be obtained by replacing one or more amino acids in SEQ ID NOs:8 to 13 with other amino acids without substantially alternating the aforesaid immunological activity, or by adding one or more amino acids to either or both ends of the amino acid sequences in SEQ ID NOs:8 to 13.

Concretely, only the amino acid sequences in SEQ ID NOs:8 to 13, which are essential for their recognition by T-cells, are not treated, but the rest of the amino acids in SEQ ID NOs:8 to 13 are replaced with other amino acids without substantially alternating their immunological activity as a T-cell epitope. Alternatively, one or more appropriate amino acids such as alanine can be coupled to either or both ends of the above amino acid sequences which are essential for T-cell recognition to form those having a length enough for T-cell recognition as a whole, i.e. 10 to 20 amino acid residues. Examples of such amino acid sequences are those in SEQ ID NOs:1 to 7, and examples of their homologous ones are peptides with the amino acid sequences in SEQ ID NOs:16 and 24.

The peptide according to the present invention can be readily prepared by conventional peptide synthesis well known in this art as "solid phase method" and "liquid phase method". The present specification does not explain the synthesis of the present peptide because the present invention in itself does not relate to the peptide synthesis. Such a technique is described in detail, for example, in the chapter "*Protein VI*" in "*Shi*-*Seikagaku-Jikken-Koza*", Vol.1, pp.3-44 (1992), edited by *The* *Japanese Biochemical Society*, Tokyo, Japan, published by Tokyo Kagaku Dojin, Tokyo, Japan. The peptide according to the present invention is not restricted to those which are chemically synthesized, and includes other peptides which are prepared from cedar pollens and cedar male flowers, and those prepared by appropriately decomposing cedar pollen allergens obtained by the recombinant DNA technology, and collecting the objective from the resultant. In addition, DNAs, which encode peptides having the amino acid sequences in SEQ ID NOs:8 to 13 and homologous ones to them, can be prepared and inserted into self-replicable vectors to form recombinant DNAs, then introduced into appropriate hosts such as *Escherichia coli*, *Bacillus*, and yeasts to obtain transformants. The present peptide can be also obtained by culturing such transformants in nutrient culture media, and collecting the peptide from the cultures. DNAs, which encode the peptides having the amino acid sequences in SEQ ID NOs:8 to 13, can be prepared based on a base sequence of a cDNA as disclosed in Japanese Patent Application No.344,596/93 and International Publication Number WO 93/01,213. The present peptide may be modified into those in the form of a conjugate which can be prepared by adding saccharides and polyethylene glycol to the present peptide, as well as those in the form of derivatives and polymers prepared by bridging and bonding the peptide by acetylation, amidation and/or polyfunctional reagents.

Although the present peptide exerts a desired therapeutic- and/or prophylactic-effect even when administered to patients in a relatively crude form, it is generally purified before use. The purification techniques usable in the present invention include those which are used in this art to purify peptides and proteins, for example, filtration, concentration, centrifugation, gel filtration chromatography, ion-exchange chromatography, high-performance liquid chromatography, affinity chromatography, gel electrophoresis and isoelectrophoresis. If necessary, two or more of these techniques can be used in combination. The resultant purified peptide may be further concentrated and/or lyophilized into products in the form of liquid or solid to meet to final use.

As is described above, the present polypeptide does not substantially react with immunoglobulin E antibody specific to cedar pollen allergens, and significantly activates T-cells specific to the allergens. Therefore, the present peptide has a wide applicability as an immunotherapeutic agent to treat and/or prevent cedar pollinosis. Immunotherapeutic agents containing the present peptide as an effective ingredient can treat cedar pollinosis without substantial induction of side effects such as anaphylaxis when administered to mammals including human with cedar pollinosis. In the case of administering the present immunotherapeutic agent to healthy human and animals with potential cedar pollinosis, it exerts a significant prophylactic efficacy on cedar pollinosis and strongly remits allergic symptoms in case of need.

Explaining now the immunotherapeutic agent according to the present invention in more detail, it usually contains one or more peptides usable in the present invention in an amount of 0.01-100 w/w %, preferably, 0.05-50 w/w %, more preferably, 0.5-5.0 w/w %, d.s.b. The immunotherapeutic agent according to the present invention includes the present peptide alone or compositions comprising the peptide and one or more carriers, diluents, excipients, adjuvants and stabilizers such as serum albumin, gelatin, mannitol, maltose and trehalose, as well as anti-inflammatory agents and antihistaminics such as a steroid hormone and sodium cromoglycate. The present immunotherapeutic agent includes those in a unit dose form which contain the present peptide in an amount of a daily dose to several folds of dose by integers, i.e. from 4 to 1/40 times, and those in the form of a physically separated systematic agent suitable for administration. Examples of such agents in a unit dose form are powders, fine granules, granules, pills, tablets, capsules, trochees, syrups, emulsions, ointments, plasters, cataplasms, suppositories, ophthalmic solutions, nasal drops, nebulae and injections.

Explaining now the administration method of the present immunotherapeutic agent, it can be administered percutaneously, orally, nasally, ophthalmically and in injection manner to mammals in general including human to treat and/or prevent cedar pollinosis. Although the dose for human is changeable depending on its purpose and patients's symptoms, it is usually administered to patients at a dose in the range of 0.01-1.0 g/day/adult, preferably, 0.01-0.1 g/day/adult once in a week or a month over a period of about 1-6 months while observing the patients' symptoms and conditions. Usually, the dose is gradually increased and administered repeatedly.

The following examples explain the preparation of the present peptide and uses thereof:

### Example A-1

### Preparation of peptide

Peptides having the amino acid sequences in SEQ ID NOs:8 to 11 were in usual manner chemically synthesized using "EXCELL®", purified on "MODEL Hi-Pore RP-318", a reverse phase high-performance liquid chromatography commercialized by Bio-Rad Laboratories Inc., Brussels, Belgium, up to give a purity of 95 w/w % or higher, d.s.b., and lyophilized into solid preparations. Each preparation was sampled and analyzed on "MODEL 470A", a peptide sequencer commercialized by Perkin-Elmer Corp., Instrument Div., Norrwalk, USA, and revealed that the synthesized four peptides had the prescribed amino acid sequences.

### Example A-2

### Preparation of peptide

Peptides having the amino acid sequences in SEQ ID NOs:12 and 13 were in usual manner chemically synthesized using "CLEAVABLE PEPTIDE KIT", and, similarly as in Example A-1, purified up to give a purity of 95 w/w %, d.s.b., and lyophilized into solid preparations. Each preparation was sampled and analyzed similarly as in Example A-1 confirming that the synthesized four peptides had the prescribed amino acid sequences.

### Example A-3

### Preparation of peptide

Similarly as in Example A-1, a peptide having the amino acid sequence in SEQ ID NO:16 was chemically synthesized and purified up to give a purity of 95 w/w %, d.s.b. Thereafter, the purified peptide was sampled and analyzed similarly as in Example A-1 revealing that it had the prescribed amino acid sequence.

### Example A-4

### Preparation of peptide

Similarly as in Example A-2, a peptide having the amino acid sequence in SEQ ID NO:17 was chemically synthesized and purified up to give a purity of 95 w/w %, d.s.b. The purified peptide was sampled and analyzed similarly as in Example A-1 revealing that it had the prescribed amino acid sequence.

### Example A-5

### Preparation of peptide

Similarly as in Examples A-1 and A-2, peptides, which have the amino acid sequences in SEQ ID NOs:18 to 24 corresponding to the samples D-1 to D-7 in Experiment 3, were chemically synthesized, purified up to give a purity of 95 w/w %, and lyophilized into a solid product. The product was sampled and analyzed on "MODEL 470A", a peptide sequencer commercialized by Perkin-Elmer Corp., Instrument Div., Norrwalk, USA, revealing that the synthesized seven peptides had the prescribed amino acid sequences.

### Example B-1

### Solution

Six peptides obtained by the methods in Examples A-1 and A-2 were respectively dissolved in distilled water containing one w/v % purified gelatin as a stabilizer to give a final concentration of 0.1 g/ml, and the solutions were in usual manner membrane filtered into six solutions.

Generally, each individual differently reacts to the peptides according to the present invention. Therefore, these six solutions may be blended in appropriate ratios into the most suitable composition for individuals. The composition has a satisfactory stability and can be arbitrarily used to treat and/or prevent cedar pollinosis as a solution for ophthalmic solutions, collunariums and oral nebulae.

### Example B-2

### Injection

Six peptides obtained by the methods in Examples A-1 and A-2 were dissolved in physiological saline containing one w/v % human serum albumin to give a respective final concentration of 0.01, 0.1 or 1 mg/ml, and the solution was membrane filtered and distributed into sterilized vials by 2 ml and lyophilized, followed by cap sealing the vials.

Before use, the product is admixed with and dissolved to homogeneity in one ml of distilled water for injection. The product, which has a satisfactory stability and contains six peptides as an effective ingredient, can be arbitrarily used as a dried injection for treating and/or preventing cedar pollinosis.

### Example B-3

### Tablet

Two g of a purified pullulan, an average molecular weight of about 20,000 daltons, was dissolved to homogeneity in 100 ml distilled water, and the solution was mixed with 2 ml of 1.7 w/v % cyanuric chloride in acetone, followed by an enzymatic reaction at 5°C for 2 hours while stirring and keeping the pH at 7 with 5 w/v % aqueous sodium carbonate solution. Thereafter, the reaction mixture was dialyzed against 4°C cold water overnight while keeping the pH at 7 similarly as above to obtain a 20 ml aqueous solution containing an activated pullulan.

To the solution were added peptides having the amino acid sequences in SEQ ID NOs:8, 10 and 11 obtained by the method in Example A-1, a peptide having an amino acid sequence in SEQ ID NO:13 obtained by the method in Example A-2, a peptide obtained by the method in Example A-3, and a peptide obtained by the method in Example A-4 in respective amounts of 0.2 mg, and the mixture solution was subjected to an enzymatic reaction at 37°C for 12 hours while gently stirring and keeping the pH at about 7. After completion of the enzymatic reaction, the reaction mixture was mixed with 4 g glycine and incubated at 37°C for 5 hours under gentle stirring conditions to block intact active groups of the peptides.

The reaction mixture thus obtained was fed to a column packed with "SEPHADEX G-50" which had been equilibrated with 0.1 M phosphate buffer (pH 7.0), and fed with a fresh preparation of the same buffer to fractionate and collect a conjugate of the present peptides and pullulan. The yield was about 30% to the material peptides, d.s.b.

The fractions containing the conjugate were pooled, membrane filtered, concentrated, lyophilized, pulverized, mixed to homogeneity with mannitol, and tabletted into tablets, 200 mg weight each, containing 2, 10 or 50 mg of the conjugate.

The product with a satisfactory absorbency and stability can be arbitrarily used as a sublingual agent for treating and/or preventing cedar pollinosis.

### Example B-4

### Syrup

One g of a purified lipopolysaccharide derived from *Escherichia coli* was dissolved in 100 ml of 10 mM aqueous calcium phosphate solution, and the solution was mixed with 6 ml of 100 mM sodium periodate and incubated at ambient temperature for 20 min to activate the lipopolysaccharide. The resultant mixture was dialyzed against one M glycine-HCl buffer (pH 4.4) at 4°C overnight to remove the remaining intact periodic acid, and adjusted to pH of about 9.5 by the addition of 0.1 M sodium bicarbonate buffer. Six peptides obtained by the methods in Examples A-1 and A-2 were dissolved in respective amounts of 10 mg in 100 ml of 0.1 M phosphate buffer (pH 7.0), and the solution was added to the mixture containing the activated lipopolysaccharide. The mixture solution thus obtained was enzymatically reacted by standing at ambient temperature for 12 hours.

The reaction mixture was purified by the method in Example B-3, and the resultant fractions containing conjugates of the peptides and the lipopolysaccharide were pooled, concentrated and pulverized into a solid product. The yield was about 30% to the material peptides, d.s.b.

The product and sucrose were dissolved in distilled water containing one w/v % purified gelatin to give final concentrations of 0.1 or 1 mg/ml and 50 w/w %, respectively, and the solution was membrane filtered in usual manner to obtain a syrupy product. The product was distributed into sterilized vials by 2 ml and the vials were cap sealed to obtain the desired product.

The product, having a satisfactory stability and containing the conjugate, can be arbitrarily used as a syrup for treating and/or preventing cedar pollinosis.

### Example B-5

### Solution

Each of seven peptides, obtained by the method in Example A-5, was dissolved in distilled water containing one w/v % purified gelatin as a stabilizer to give a final concentration of 0.1 g/ml, and sterilized by filtration in usual manner to obtain seven solutions.

The susceptibility to the present polypeptide is usually changeable depending on individuals to be administered, so that the desired product is prepared by mixing the above seven solutions to give the most suitable ratio. The product thus obtained has a satisfactory stability and can be arbitrarily used as a solution for ophthalmic solutions, collunariums, and oral nebulae to treat and/or prevent cedar pollinosis.

### Example B-6

### Injection

Seven peptides obtained in EXample A-5 were dissolved in physiological saline containing one w/v % human serum albumin as a stabilizer to give a respective final concentration of 0.01, 0.1 or 1 mg/ml, sterilized by filtration, distributed to sterilized vials by 2 ml, lyophilized and cap sealed.

Prior to use, the product is admixed with one ml/vial of distilled water for injection, and the content is dissolved to homogeneity. The product, which has the seven peptides as an effective ingredient and a satisfactory stability, can be arbitrarily used as a dried injection for treating and/or preventing cedar pollinosis.

### Example B-7

### Syrup

In distilled water, containing one w/v % purified gelatin, were dissolved the seven peptides, obtained by the method in Example A-5, to give a respective concentration of 0.1 mg/ml, and further dissolved sucrose to give a concentration of 50 w/v %. The resultant solution was sterilized by filtration into a syrup which was then distributed to sterilized vials by 2 ml, and cap sealed to obtain a desired product.

The product, which has a satisfactory stability and contains the peptides according to the present invention, can be arbitrarily used as a syrup for treating and/or preventing cedar pollinosis.

As is described above, the present invention was made based on the finding of a peptide essentially consisting of T-cell epitopes of cedar pollen allergens. The peptide does not substantially react with immunoglobulin E antibody which is specific to cedar pollen allergens, and activates T-cells specific to cedar pollen allergens without substantial induction of anaphylaxis when administered to mammals including human. Thus, the present immunotherapeutic agent which contains the peptide as an effective ingredient exerts a satisfactory efficacy on the treatment and/or prevention of cedar pollinosis in a relatively short period of time without fear of causing side effects when administered to mammals including human. The present peptide with a strong safeness can be used in the treatment and/or the prevention of cedar pollinosis because it is readily preparable in a desired amount, and the quality control is readily feasible.

The present invention with these satisfactory effects and functions does greatly contribute to this field.

While there has been described what is at present considered to be the preferred embodiments of the invention, it will be understood the various modifications may be made therein, and it is intended to cover in the appended claims all such modifications as fall within the true spirit and scope of the invention.

## Claims

1. A peptide which does not substantially react with immunoglobulin E antibody specific to a cedar pollen allergen, and significantly more activates T-cells specific to the allergen than a negative control when assayed by the ³H-thymidine uptake method.

2. The peptide of claim 1, which consists of 5-50 amino acids.

3. The peptide of claim 1, which contains either amino acid sequence of SEQ ID NOs:1 to 7.

4. The peptide of claim 1, which contains either amino acid sequence of SEQ ID NOs:8 to 13.

5. The peptide of claim 1, which has an amino acid sequence which is homologous to either one of SEQ ID NOs:8 to 13.

6. The peptide of claim 5, which has either amino acid sequence of SEQ ID NOs:14 and 15.

7. The peptide of claim 1, which is obtained by adding one or more amino acids to either amino acid sequence of SEQ ID NOs:1 to 7 without substantially changing the properties of said peptide as specified in claim 1.

8. The peptide of claim 7, which has either amino acid sequence of SEQ ID NOs:18 to 24.

9. The peptide as claimed in claim 1, which originates from a cedar pollen allergen preparable by:
(a) collecting a cedar pollen from a male flower of *Cryptomeria japonica*;
(b) soaking the cedar pollen in an aqueous solution at pH 8.2 for extraction;
(c) preparing from the resultant extract an aqueous solution containing cedar pollen allergens;
(d) subjecting the solution to DEAE-Sephadex and CM-Sephadex column chromatographies in the given order;
(e) collecting and subjecting the resultant fractions from CM-Sephadex column which contain cedar pollen allergens to Mono-S column chromatography using a linear gradient buffer of sodium chloride increasing from 0 M to 0.5 M for sodium chloride; and
(f) collecting the resultant fractions eluted at around 0.4 M of sodium chloride.

10. The peptide as claimed in claim 1, which originates from a cedar pollen allergen preparable by:
(a) collecting a cedar pollen from a male flower of *Cryptomeria japonica*;
(b) soaking the cedar pollen in an aqueous solution at pH 8.2 for extraction;
(c) preparing from the resultant extract an aqueous solution containing cedar pollen allergens;
(d) subjecting the solution to DEAE-Sephadex and CM-Sephadex column chromatographies in the given order;
(e) collecting and subjecting the resultant fractions from CM-Sephadex column which contain cedar pollen allergens to Mono-S column chromatography using a linear gradient buffer of sodium chloride increasing from 0 M to 0.5 M for sodium chloride; and
(f) collecting the resultant fractions eluted at around 0.1 to 0.3 M of sodium chloride.

11. An amino acid sequence of SEQ ID NO:1 of said peptide as claimed in claim 1.

12. An amino acid sequence of SEQ ID NO:2 of said peptide as claimed in claim 1.

13. An amino acid sequence of SEQ ID NO:3 of said peptide as claimed in claim 1.

14. An amino acid sequence of SEQ ID NO:4 of said peptide as claimed in claim 1.

15. An amino acid sequence of SEQ ID NO:5 of said peptide as claimed in claim 1.

16. An amino acid sequence of SEQ ID NO:6 of said peptide as claimed in claim 1.

17. An amino acid sequence of SEQ ID NO:7 of said peptide as claimed in claim 1.

18. An immunotherapeutic agent which contains said peptide of claim 1 as an effective ingredient.

19. The agent of claim 18, wherein said peptide consists of 5-50 amino acids.

20. The agent of claim 18, which contains two or more peptides of claim 1.

21. The agent of claim 18, wherein said peptide contains either amino acid sequence of SEQ ID NOs:1 to 7.

22. The agent of claim 18, wherein said peptide contains either amino acid sequence of SEQ ID NOs:8 to 13.

23. The agent of claim 18, wherein said peptide has an amino acid sequence which is homologous to either one of SEQ ID NOs:8 to 13.

24. The agent of claim 23, wherein said peptide has either amino acid sequence of SEQ ID NOs:14 and 15.

25. The agent of claim 18, wherein said peptide is obtained by adding one or more amino acids to either amino acid sequence of SEQ ID NOs:1 to 7 without substantially changing the properties as specified in claim 1.

26. The agent of claim 18, wherein said peptide has either amino acid sequence of SEQ ID NOs:18 to 24.

27. The agent of claim 1, wherein said peptide originates from a cedar pollen allergen preparable by:
(a) collect ing a cedar pollen from a male flower of *Cryptomeria japonica;*
(b) soaking the cedar pollen in an aqueous solution at pH 8.2 for extraction;
(c) preparing from the resultant extract an aqueous solution containing cedar pollen allergens;
(d) subjecting the solution to DEAE-Sephadex and CM-Sephadex column chromatographies in the given order;
(e) collecting and subjecting the resultant fractions from CM-Sephadex column which contain cedar pollen allergens to Mono-S column chromatography under a linear gradient buffer of sodium chloride increasing from 0 M to 0.5 M for sodium chloride; and
(f) collecting the resultant fractions eluted at around 0.4 M of sodium chloride.

28. The agent of claim 18, wherein said peptide originates from a cedar pollen allergen preparable by:
(a) collecting a cedar pollen from a male flower of *Cryptomeria japonica*;
(b) soaking the cedar pollen in an aqueous solution at pH 8.2 for extraction;
(c) preparing from the resultant extract an aqueous solution containing cedar pollen allergens;
(d) subjecting the solution to DEAE-Sephadex and CM-Sephadex column chromatographies in the given order;
(e) collecting and subjecting the resultant fractions from CM-Sephadex column which contain cedar pollen allergens to Mono-S column chromatography using a linear gradient buffer of sodium chloride increasing from 0 M to 0.5 M for sodium chloride; and
(f) collecting the resultant fractions eluted at around 0.1 to 0.3 M of sodium chloride.

29. The agent of claim 18, wherein the content of said peptide is 0.01-100 w/w %, on a dry solid basis.

30. The agent of claim 18, which contains a member selected from the group consisting of serum albumin, gelatin, mannitol, maltose, trehalose, and mixtures thereof as a stabilizer or an excipient.
